# EUROPEAN PATENT APPLICATION

(11) **EP 2 647 396 A1**
(43) Date of publication of application: **09.10.2013**
(21) Application number: 12183236.4
(22) Date of filing: 05.09.2012
(51) Int. Cl.: A61M 1/00

(54) **Negative pressure wound therapy pump with tilt and fill sensors**

(30) Priority: 05.04.2012 US 201261620616 P; 13.08.2012 US 201213584441
(71) Applicant: Oakwell Distribution, Inc., King of Prussia, PA 19406 (US)
(72) Inventor: Li, Pan, Arcadia, CA California 91006 (US); Li, Jiang, Kunshan (CN); Lin, Huiyong, Kunshan (CN); Yang, Tongfeng, Kunshan (CN)
(74) Representative: Harrison IP Limited

(57) **Abstract**

A pump (10) includes a canister (20) for collecting fluids from a negative pressure wound therapy bandage (34) in which the canister (20) incorporates both tilt (39) and fill (38) sensors operatively connected to a microprocessor (51) to control the operation of the pump (10) in drawing fluids from the negative pressure bandage (34). The fill sensors (38) include a pair of spaced sensor pins that convey a filled signal when fluid within the canister (20) interconnects the two sensor pins. The tilt sensor (39) is housed within the pump housing (12). The canister (20) is also formed with an optical sensor (30) that provides an indication of proper alignment of the canister (20) on the pump housing (12). The optical sensor (30) includes a reflector (33) in the canister (20) that reflects an infrared light emanating from the pump housing (12). When the light reflection is received properly within the pump housing (12), the canister (20) is properly aligned and mounted on the pump housing (12).

## Description

### FIELD OF THE INVENTION

The present invention relates generally to negative pressure wound therapy system and, more particularly, to an exudates canister co-operable with a pump providing a source of negative pressure for the bandage system to collect the exudates and fluid extracted from the negative pressure bandage.

### BACKGROUND OF THE INVENTION

Negative pressure wound therapy involves a bandage system that is applied to the wound site on the patient to create a seal around the perimeter of the bandage system and around a periphery of the wound to be treated. The negative pressure bandage system is provided with a connector that connects to a pump that draws a vacuum on the bandage system to urge any fluid and exudates within the wound site to move toward the pump through a conduit interconnecting the connector and the pump. A canister is connected to the conduit to intercept the fluids and exudates before reaching the pump to collect the fluids and exudates until the canister is filled to a predetermined level. Preferably, the canister can be removed from the pump housing and replaced when filled.

In U. S. Patent No. 6,139,982, granted to Kenneth W. Hunt, et al on November 7, 2000, a negative pressure wound therapy apparatus is disclosed in which a canister is removably mounted in a pump housing and connected by a conduit to the pump to draw a vacuum on the canister. A separate conduit connects the canister to the negative pressure bandage system to draw the fluids and exudates from the wound being treated into the canister. A filter is provided at the outlet end of the canister where the conduit interconnecting the canister and the pump is located to prevent the introduction of the fluids and exudates collected into the canister from the bandage system into the pump.

U. S. Patent No. 7,004,915, issued to Thomas A. Boynton, et al on February 28, 2006, discloses a canister that is connected by a first conduit to the negative pressure bandage system and by a second conduit to the pump that asserts a negative pressure on the canister through the second conduit, which vacuum is asserted through the canister to the first conduit and the connected bandage system. The canister incorporates first and second hydrophobic filters at the connection of the second conduit to the canister such that the first hydrophobic is adapted to operate as a fill sensor for the canister and the second hydrophobic filter further inhibits contamination of the pump by the collected fluids and exudates from the wound site. An odor filter is also provided between the first and second hydrophobic filters to counteract the production of malodorous vapors present in the collected wound exudates.

In U. S. Patent No. 7,611,500, granted on November 3, 2009, to Cesar Z. Lina, et al, the canister includes an outlet that is plugged onto a port supported on the pump housing to connect the canister with the vacuum source. A switch carried on the pump housing closes when the canister is properly seated on the port. The canister incorporates a filter cap that allows the pump to draw air from the canister through the port and assert a vacuum on the negative pressure bandage system. The canister also incorporates a fill sensor in the form of a capacitive sensor that identifies a change in capacitance within the canister corresponding to the fluid level reaching the fill sensor located on the side of the canister near the outlet.

In each of the above-described prior art canisters, the fluids and exudates are drawn from the negative pressure bandage directly into the canister where the fluids and exudates are collected. Typically, the movement of the fluids and exudates is restricted from contaminating the pump by a hydrophobic filter that prevents the fluids and exudates from entering the vacuum line to the pump. The canister is preferably removable from the pump housing and disposed when filled, to be replaced by a new canister. With fill sensors specifically located on the canister, orientation of the canister is highly critical to prevent the fluids from being sensed by the fill sensor.

Many known negative pressure wound therapy systems commercially available are portable devices, meaning that the pump and the canister are sufficiently small as to be capable of being attached to the patient and moved from one location to another as the patient moves about. To ensure that the fluid and exudates that have been removed from the wound site are not able to flow back into the wound site, or back to the bandage over the wound site, canisters are often provided with tilt sensors that are operably connected to the pump and determine the orientation of the canister. When the angle of tilt exceeds a certain allowed maximum, the operation of the vacuum pump is terminated.

### OBJECT OF THE INVENTION

It would be desirable to provide a fluid and exudates collection system that is less dependent on orientation of the canister to operate properly. It would also be desirable to provide sensors that would accurately reflect the filling of the canister no matter how the canister is oriented with respect to vertical.

### SUMMARY OF THE INVENTION

According to a first aspect of the invention, there is provided a pump providing a vacuum to a negative pressure wound therapy bandage, said pump including a housing on which is mounted a removable canister for storing fluids and exudates received from said bandage, comprising:
a fill sensor operatively associated with said canister to provide a fill signal indicative of fluid within said canister reaching said fill sensor;
a tilt sensor operatively associated with said pump to provide a tilt signal indicative of said pump being positioned in a non-vertical orientation;
a microprocessor control operatively connected to said tilt sensor and fill sensor to receive said fill and tilt signals therefrom; and
said microprocessor controlling the operation of said pump in response to said tilt and fill signals.

According to a second aspect of the invention, there is provided a method of controlling the operation of a pump providing a vacuum to a negative pressure wound therapy bandage, said pump including a housing mounting a detachable canister for storing fluids and exudates received from said bandage, comprising the steps of:
providing a microprocessor control for said pump;
generating a fill signal from a fill sensor indicating the level of fluid within said canister has reached said fill sensor, said fill signal being received by said microprocessor;
generating a tilt signal from a tilt sensor indicating the positioning of said pump in a non-vertical orientation, said tilt signal being received by said microprocessor; and
deactivating the operation of the pump when said fill signal is received without said tilt signal.

In accordance with the further aspect, the invention provides a pump coupled with a medical apparatus in which fluids are received in a canister mounted on a pump housing, comprising:
a microprocessor controlling the operation of said pump in response to receipt of tilt and fill signals;
a fill sensor operatively associated with said canister to provide said fill signal to said microprocessor indicative of fluid within said canister reaching said fill sensor;
a tilt sensor operatively associated with said pump to provide said tilt signal to said microprocessor indicative of said pump being positioned in a non-vertical orientation; and
an optical sensor operatively interconnecting said pump housing and said canister to provide an indication of said canister being properly mounted on said pump housing.

The optical sensor nay suitably comprise a light source supported on said pump housing; and a reflector supported on said canister to reflect light back to said light source to provide said indication of proper mounting of the canister on the pump.

The microprocessor may deactivate -powers the pump when one of the following conditions exists, namely :
when said fill signal is received without said tilt signal; and
after a second delay when the fill signal continues to be received with the tilt signal.

Embodiments of the invention, as described herein, provide a pump for use with negative pressure wound therapy systems and having tilt and fill sensors that indicate when the pump canister is filled with fluid from the wound. Such sensors may be provided on a pump having a canister for collecting fluids from a wound being treated with a negative pressure bandage.

It is a feature of embodiments of the invention that a microprocessor is operatively coupled with the tilt and fill sensors to avoid a false indication of a full canister.

### BRIEF DESCRIPTION OF THE DRAWINGS

The invention will now be described further, by way of example, with reference to the accompanying drawings, in which:
Fig. 1 is an exploded front perspective view of a negative pressure wound system pump and canister incorporating the principles of the instant invention;
Fig. 2 is an exploded rear perspective view of the negative pressure wound system pump and canister shown in Fig. 1;
Fig. 3 is a left side perspective view of the pump and canister shown in Figs. 1 and 2, but depicting the initial engagement of the canister onto the pump housing;
Fig. 4 is a left side perspective view of the pump and canister shown in Fig. 3, but having the canister about to patch onto the pump housing;
Fig. 5 is a front perspective view of a negative pressure wound system with the assembled pump and canister connected to a negative pressure bandage;
Fig. 6 is a schematic front elevational view of the canister showing a vertical orientation with the canister fill sensors indicating a filled condition;
Fig. 7 is a schematic side elevational view of the canister shown in Fig. 6;
Fig. 8 is a schematic front elevational view of the canister tilted to the right side at an angle of 30 degrees with the canister fill sensors indicating a filled condition;
Fig. 9 is a schematic front elevational view of the canister tilted to the right side at an angle of 75 degrees with the canister fill sensors indicating a filled condition;
Fig. 10 is a schematic front elevational view of the canister tilted to the left side at an angle of 30 degrees with the canister fill sensors indicating a filled condition;
Fig. 11 is a schematic front elevational view of the canister tilted to the left side at an angle of 75 degrees with the canister fill sensors indicating a filled condition;
Fig. 12 is a schematic left side elevational view of the canister tilted backward at an angle of 30 degrees with the canister fill sensors indicating a filled condition;
Fig. 13 is a schematic left side elevational view of the canister tilted backward at an angle of 75 degrees with the canister fill sensors indicating a filled condition;
Fig. 14 is a schematic left side elevational view of the canister tilted forward at an angle of 30 degrees with the canister fill sensors indicating a filled condition;
Fig. 15 is a schematic left side elevational view of the canister tilted forward at an angle of 75 degrees with the canister fill sensors indicating a filled condition;
Fig. 16 is a logic flow diagram reflecting the operation of the tilt and level sensors in the control of the pump; and
Fig. 17 is a schematic block diagram representing the control logic functions.

### DETAILED DESCRIPTION OF THE PREFERRED EMBODIMENT

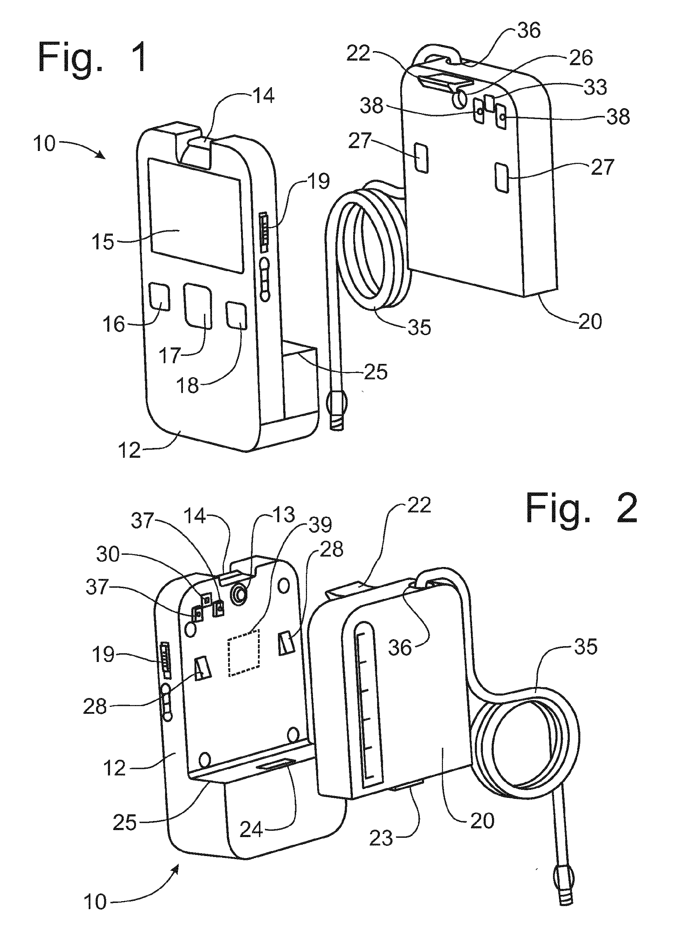

Referring to Figs. 1 - 5, a pump for a negative pressure wound therapy system can best be seen. The pump 10 is mounted in a pump housing 12 that draws a vacuum from the vacuum port 13 for the purposes of extracting fluids and exudates from a negative pressure bandage 34, as will be discussed in greater detail below. The pump housing 12 is provided with a display screen 15 and control buttons 16 - 19 for powering the operation of the pump and monitoring the function thereof. The top surface of the pump housing 12 is formed with a latch keeper 14 to retain the canister 20 on the pump housing in operative communication therewith, as will also be described in greater detail below. In the way of examples, the control buttons 16 - 19 can provide operations control for the pump 10. The control button 16 can be used to set the operating pressure for the pump 10. Control button 17 can be used to turn the pump 10 on and off to start or stop the negative pressure therapy. Control button 18 can define the mode of operation, such as continuous or intermittent operation of the pump 10. Control switch 19 can be used to turn the electronics on and off, the powering of the electronics being necessary before the other control buttons 16 - 18 can be operated.

The canister 20 is a hollow structure for collecting and storing the fluids and exudates extracted from the negative wound therapy bandage 34. The canister 20 is detachably supported on the mounting ledge 25 of the pump housing 12 and operatively cooperable therewith to receive a vacuum therefrom and to apply that vacuum to the negative pressure bandage 34 to extract fluids and exudates therefrom. The canister 20 is formed with a latch member 22 at the upper edge thereof to be positionable for engagement with the latch keeper 14 on the pump housing 12. Also, the bottom surface of the canister 20 is formed with a mounting tab 23 that is sized to insert into a positioning slot 24 formed in the housing ledge 25 to secure the canister 20 on the pump housing 12 and to assure that the canister 20 is properly mounted on the pump housing 12.

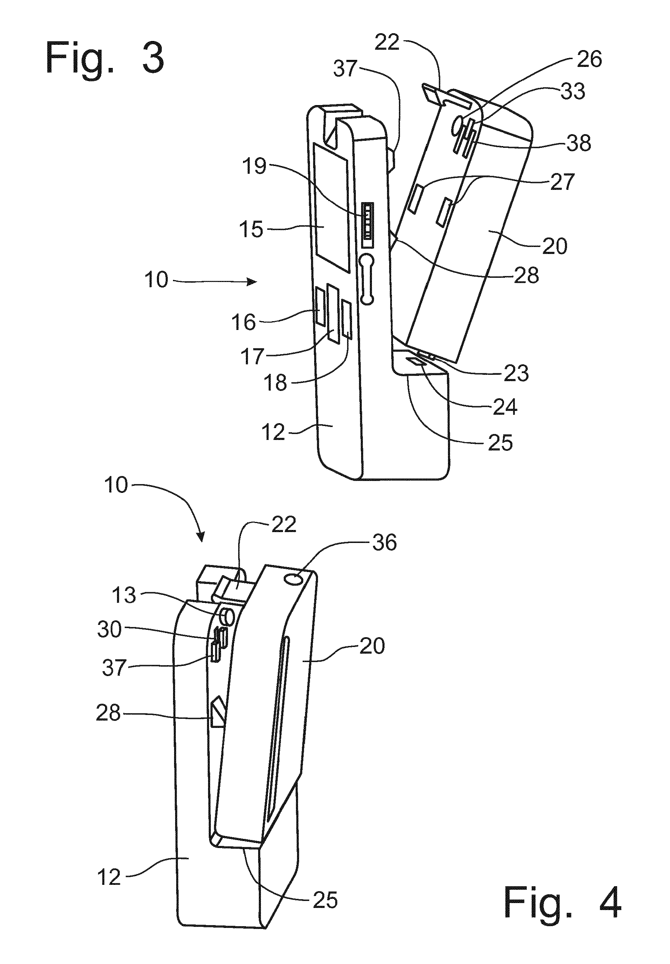

The canister 20 is provided with a receiver port 26 that is aligned with the vacuum port 13 when the canister 20 is properly mounted on the pump housing 12 so that the pump 10 can draw a vacuum on the canister 20. The canister 20 is also provided with retainer holes 27 that receive retainer tabs 28 formed on the pump housing 12 to stabilize the positioning of the canister 20 on the pump housing 12. The process to mount the canister 20 on the pump housing 12 is shown in Figs. 3 and 4. The canister 20 is first positioned on the ledge 25 of the pump housing 12 so that the mounting tab 23 slides into the corresponding positioning slot 24. The canister 20 is then rotated about the mounting tab 23 until the latch 22 snaps over the latch keeper 14 to secure the canister 20 onto the pump housing 12. If the canister 20 is properly aligned, the retainer tabs 28 will fit into the corresponding retainer holes 27 to provide lateral stability for the canister 20 relative to the pump housing 12.

Because of the required connection of the vacuum port 13 within the receiver port 26 to enable proper operation of the negative pressure wound therapy system, the pump housing 12 is provided with an optical sensor 30 that directs an infrared light onto a reflector 33 mounted on the canister 20. If the reflector 33 is not properly aligned, i.e. perpendicular to the optical sensor 30, the infrared light beam will not be reflected back into the infrared optical sensor 30. The pump 10 is operably connected to the optical sensor 30 such that the receipt of a return signal from the reflector 33 is required in order for the pump 10 to be activated. Preferably, the optical sensor 30 will initiate a message on the display screen 15 to alert the user that the canister 20 is or is not properly aligned for operation of the pump 10.

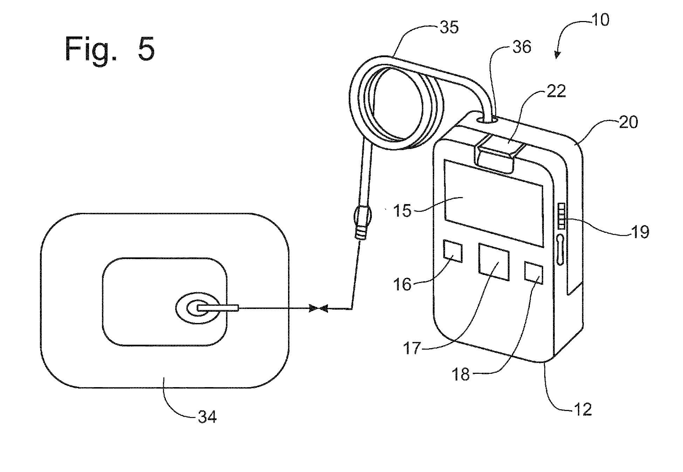

Once the canister 20 is properly seated on the pump housing 12, the pump 10 is free to operate and draw a vacuum through the vacuum port 13 engaged with the receiver port 26 into the canister 20, which is turn is applied to the tubing 35 connected to the inlet port 36 of the canister 20 and extending to the negative pressure bandage 34, as is shown in Fig. 5. Fluids and exudates are drawn into the canister 20 via the tubing 35 and fall to the bottom of the canister 20. A hydrophobic filter (not shown) is preferably utilized on the interior side of the receiver port 26 to prevent the fluids and exudates from entering into the pump 10 via the vacuum port 13.

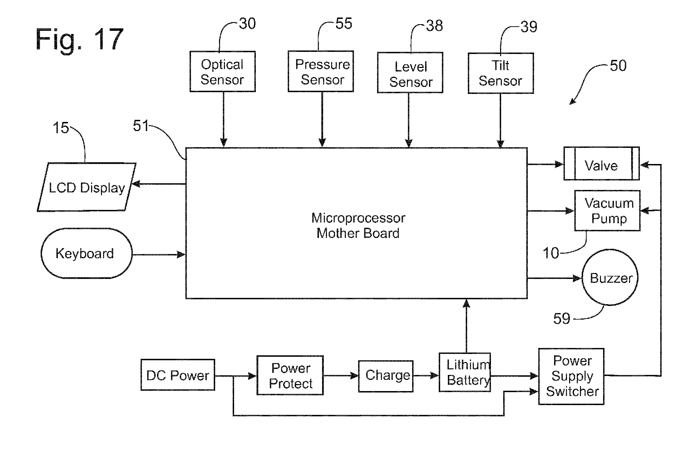

The canister 20 is provided with a pair of resister-type fill sensors 38 that project into the interior of the canister 20 and are connected to the microprocessor 51 mounted in the pump housing 12 via the contacts 37, as is depicted in the schematic block diagram of Fig. 17. The fill sensors 38 are positioned adjacent the optical sensor 30 and the reflector 33 and provide a signal to the microprocessor 51 that fluid is cross-connecting the two fill sensors 38 which allows electrical current to cross from one fill sensor 38 to the other. The completion of that electrical circuit signals the microprocessor 51 that the fill sensors 38 are being engaged by fluid within the canister 20. In addition, the pump housing 12 supports a tilt sensor 39 that can determine the direction and the angle at which the pump housing 12, and therefore the canister 20, is oriented. The signals from both the tilt sensor 39 and the fill sensors 38 are sent to the microprocessor 51 to control the operative function of the pump 10.

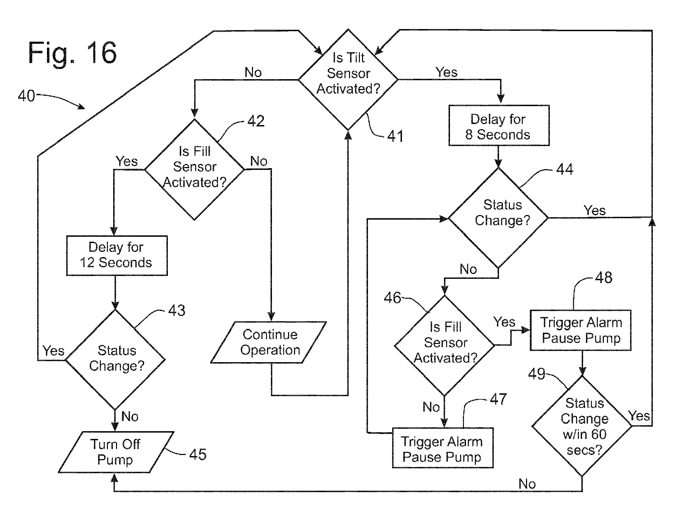

As can be seen in Fig. 16, the combination of the signals from the fill and tilt sensors 38, 39, will control the operation of the pump 10. The negative pressure therapy system, specifically the pump 10 and canister 20, will work most efficiently when the canister is oriented in an upright position. Thus, when the canister 20 is not in the upright position, the user needs to be informed of the inappropriate orientation so that the user can correct the orientation of the canister 20. Preferably, the tilt sensor 39 will be able to ascertain the number of degrees of the tilt, but will have some latitude with respect to accuracy. For example, identifying the canister 20 at a vertical orientation can encompass a vertical orientation plus or minus a few degrees.

As shown in Fig. 16, the process 40 begins at step 41 with a query as to whether the tilt sensor 39 is activated. If the tilt sensor 39 is not activated, the next query at step 42 defines whether the fill sensor 38 has been activated. If the fill sensor has not been activated, the operation of the pump 10 would continue as intended. If the fill sensor 38 has been activated at step 42, the process is delayed for about twelve seconds and then at step 43 to provide assurance that the fill sensors 38 are not being activated by a splashing of the fluids within the canister 20, which would present a false alarm. After the delay circuit is exhausted, the process 40 queries at step 43 whether either the fill sensor 38 or tilt sensor 39 status has changed. If no change in status is ascertained at step 43, then the pump 10 is turned off automatically at step 45 as the canister 20 is full. If the status at step 43 has changed, the process starts again at step 41.

If at step 41, the tilt sensor 39 has been activated, the process delays activity for eight seconds to provide a safeguard against a false signal due to movement of the canister 20 splashing fluids onto the fill sensors 38. Then at step 44, the process queries whether the tilt sensor 38 has undergone a status change. If at step 44 the tilt sensor 39 has a changed status, the process returns to step 41 to query if the tilt sensor 39 has been activated. If the response to the query at step 44 is in the negative, the process 40 queries the fill sensor 38 at step 46 to see if the fill sensor 38 has been activated. If the fill sensor 38 has not been activated, the process triggers an alarm, preferably both audible and visual, at step 47, to inform the user to reorient the canister 20, while the operation of the pump is paused until the canister has been returned to a vertical orientation.

The process 40 then returns to step 44 to see if the status of the tilt sensor 39 has changed. The alarm will not be disengaged nor the pump returned to operation until the status of the tilt sensor 39 has changed at step 44. If at step 46 the fill sensor 38 has been activated, the alarm is also triggered and the operation of the pump 10 is paused. If after sixty seconds at step 49 the status of the fill sensor has changed, then the process returns to step 41 to determine if the tilt sensor 38 is still activated. If at step 49 both the fill and tilt sensors 38, 39 remain activated, then the process will automatically shut down the pump 10 at step 45.

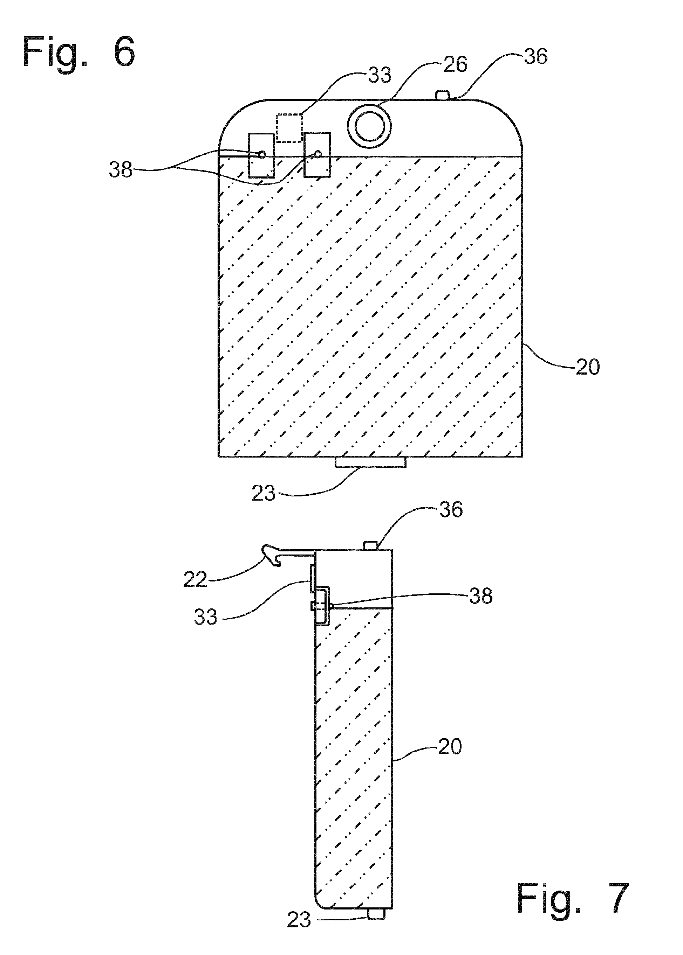

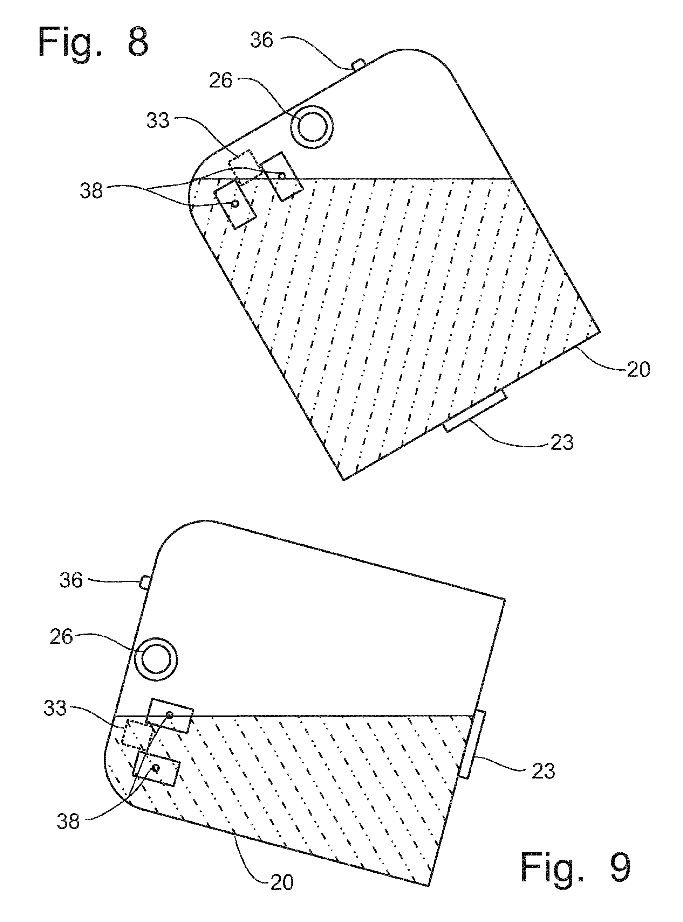

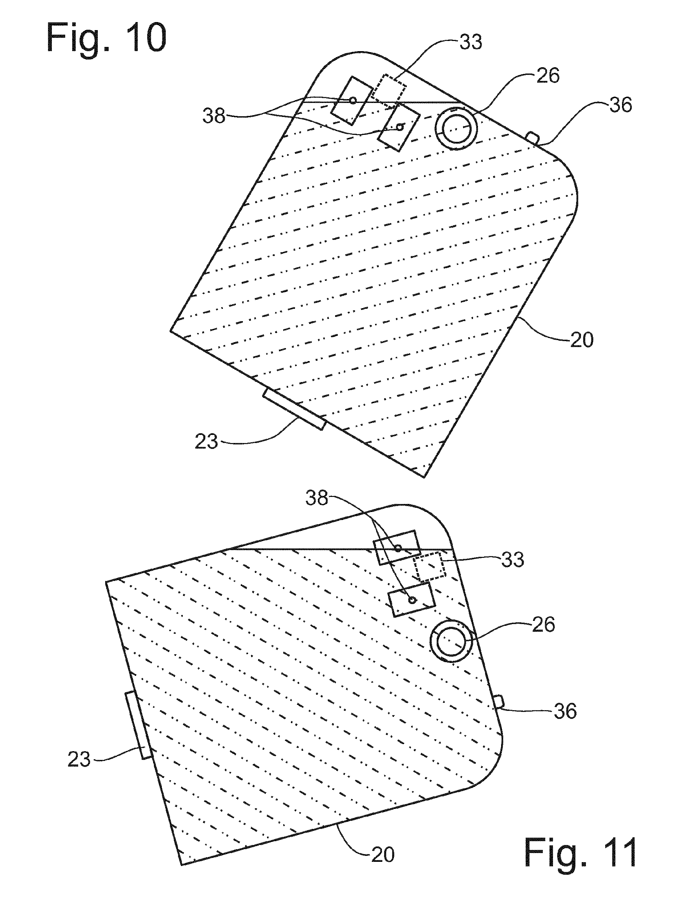

The impact of the fluid content within the canister 20 when the canister 20 is tilted in various directions is depicted in Figs. 6 - 15. The canister 20 is sized to retain approximately 111.5 ml of fluid when the canister 20 is oriented vertically, as is depicted in Figs. 6 and 7. However, when the canister 20 is tilted 30 degrees to the right, as is depicted in Fig. 8, the volume of fluid required to activate the fill sensors 38 is 99.2 ml. At a tilt angle to the right of 75 degrees, as shown in Fig. 9, the volume of fluid needed to activate the fill sensors 38 is only 55.7 ml. Conversely, a tilting of the canister 20 to the left by 30 degrees, as is depicted in Fig. 10, will enable the canister 20 to retain 122.3 ml to activate the fill sensors 38. At a left tilt angle of 75 degrees, as reflected in Fig. 11, the volume of fluid required to activate the fill sensors 38 is 121.6 ml. Accordingly, the microprocessor 51 must monitor both the tilt and fill sensor signals so that the canister 20 does not over fill.

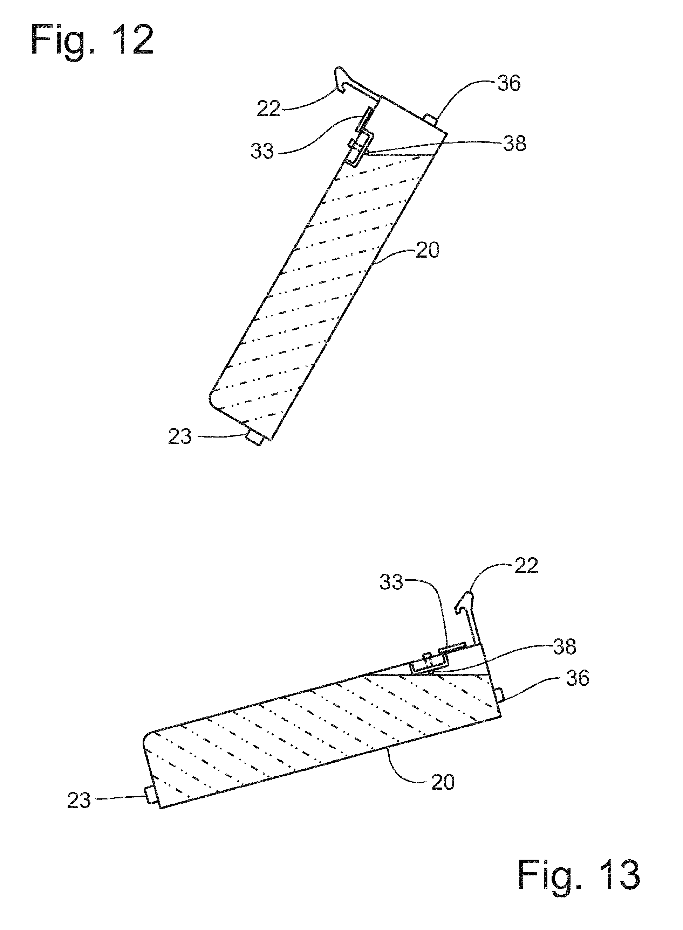

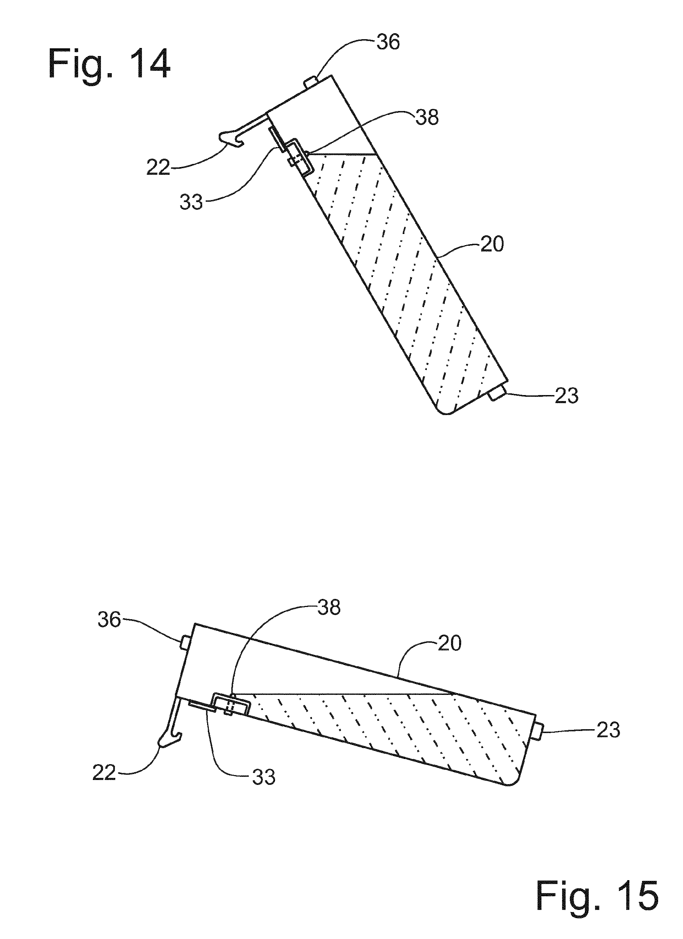

Forward and rearward tilt angles are shown in Figs. 12 - 15. In Fig. 12, the canister 20 is tilted rearward by 30 degrees, resulting in 116.0 ml of fluid needed to activate the fill sensors 38. Tilting the canister 20 backwards by 75 degrees, as shown in Fig. 13, requires 122.6 ml to activate the fill sensors 38. Tilting the canister 20 forwardly by 30 degrees, as shown in Fig. 14, requires 116.7 ml of fluid to activate the fill sensors 38, while a forward tilt of 75 degrees, as shown in Fig. 15, reduces the fluid volume to 80.8 ml to activate the fill sensors 38.

Referring now to the schematic diagram of the control logic in Fig. 17, one skilled in the art can see that the microprocessor 51 receives input from the fill and tilt sensors 38, 39, to control the continued operation of the pump 10 in the manner described above. Furthermore, the optical sensor 30 is connected to the microprocessor 51 to control the initial start up of the pump 10. Without the confirmation signal from the optical sensor 30, the microprocessor 51 will not allow the pump 10 to start operation. The microprocessor 51 also receives confirmation signals from a pressure sensor 55 to monitor the negative pressure asserted through the vacuum port 13. If the pressure rises or falls significantly, the pump 10 will also cease operating and provide a message to the user by the display screen 15 to inform the user of a pressure problem, which could be caused by a failure of the pump 10, a plugged tubing 35, or an overfilled canister 20, among other things. The visual display of an alarm or of an error message or the like, is provided to the user via the LCD display screen 15, while the auditory alarm or signal is provided via a buzzer 59 operatively coupled to the microprocessor 51.

It will be noted from the above description that embodiments of the invention offer the following advantages:
- The negative pressure wound therapy apparatus is less dependent on orientation of the pump for operation.
- The person being treated with negative pressure wound therapy is more capable of being mobile while being treated.
- The tilt sensor can be utilized to provide an indication that a triggering of the fill sensor is not indicative of a filled canister.
- The microprocessor ceases operation of the pump when the microprocessor receives a signal from the fill sensor without a signal from the tilt sensor.
- The microprocessor can sound an alarm when the tilt sensor is activated.
- The microprocessor can cease operation of the pump when both the tilt and fill sensors are received.
- The pump that provides a vacuum to a negative pressure wound therapy bandage is durable in construction, carefree of maintenance, and simple and effective in use.

## Claims

1. A pump providing a vacuum to a negative pressure wound therapy bandage, said pump (10) including a housing (12) on which is mounted a removable canister (20) for storing fluids and exudates received from said bandage, comprising:
a fill sensor (38) operatively associated with said canister (20) to provide a fill signal indicative of fluid within said canister reaching said fill sensor;
a tilt sensor (39) operatively associated with said pump (10) to provide a tilt signal indicative of said pump being positioned in a non-vertical orientation;
a microprocessor control (51) operatively connected to said tilt sensor (39) and fill sensor (38) to receive said fill and tilt signals therefrom; and
said microprocessor (51) controlling the operation of said pump (10) in response to said tilt and fill signals.

2. A pump as claimed in Claim 1 wherein said microprocessor (51) is operative to turn off the pump (10) when said fill signal is received without said tilt signal.

3. A pump as claimed in Claim 2 wherein said microprocessor (51) is operative to delay turning off the pump when said fill signal is received without said tilt signal, to determine if the status of the receipt of the fill and tilt signals changes.

4. A pump as claimed in Claim 3 wherein the turning off of the pump is delayed by a duration of about twelve seconds.

5. A pump as claimed in Claim 1 wherein said microprocessor pauses the operation of the pump after a delay when said tilt signal is received.

6. A pump as claimed in Claim 5 wherein said microprocessor turns off the pump after a second delay when the fill signal continues to be received with the tilt signal.

7. A pump as claimed in Claim 6 wherein said second delay has a duration of about sixty seconds.

8. A pump as claimed in Claim 5 wherein said microprocessor triggers an alarm with the pausing of the operation of the pump.

9. A pump as claimed in any preceding claim, wherein said canister includes an optical sensor to provide an indication of proper mounting of the canister to the pump.

10. A pump as claimed in Claim 9 wherein said optical sensor comprises:
a light source mounted on said pump; and
a reflector supported on said canister to reflect light back to said light source to provide said indication of proper mounting of the canister on the pump.

11. A method of controlling the operation of a pump providing a vacuum to a negative pressure wound therapy bandage, said pump including a housing mounting a detachable canister for storing fluids and exudates received from said bandage, comprising the steps of:
providing a microprocessor control for said pump;
generating a fill signal from a fill sensor indicating the level of fluid within said canister has reached said fill sensor, said fill signal being received by said microprocessor;
generating a tilt signal from a tilt sensor indicating the positioning of said pump in a non-vertical orientation, said tilt signal being received by said microprocessor; and
deactivating the operation of the pump when said fill signal is received without said tilt signal.

12. A method as claimed in The method of Claim 11 wherein said depowering step is accomplished after a delay to determine if the status of the receipt of the fill signal has changed.

13. A method as claimed in Claim 12 further comprising the steps of:
pausing the operation of said pump when said tilt signal is received; and
delaying said pausing step for a first period of time to ascertain if said tilt signal is still being received by said microprocessor.

14. A method as claimed in Claim 13 wherein said pausing step is accompanied by the step of triggering an alarm.

15. A method as claimed of Claim 13 wherein said microprocessor deactivates the operation of the pump when both the tilt and fill signals are received and after a second delay to ascertain if said tilt and fill signals are still being received by said microprocessor.
